# EUROPEAN PATENT SPECIFICATION

(11) **EP 4 284 492 B1**
(45) Date of publication and mention of the grant of the patent: **29.04.2026**
(21) Application number: 22713046.5
(22) Date of filing: 19.03.2022
(51) Int. Cl.: A61N 1/36

(54) **TRANSDUCER APPARATUSES FOR DELIVERING TUMOR TREATING FIELDS TO A SUBJECT'S BODY**
WANDLERVORRICHTUNGEN ZUR ABGABE VON TUMORBEHANDLUNGSFELDERN AN DEN KÖRPER EINER PERSON
APPAREILS TRANSDUCTEURS POUR ADMINISTRER DES CHAMPS DE TRAITEMENT DE TUMEUR AU CORPS D'UN SUJET

(30) Priority: 23.03.2021 US 202163164957 P; 12.08.2021 US 202163232329 P; 18.03.2022 US 202217698457
(43) Date of publication of application: 06.12.2023
(73) Proprietor: Novocure GmbH, 6340 Baar (CH)
(72) Inventor: MARCIANO, Tal, 31905 Haifa (IL); ARVATZ, Smadar, 31905 Haifa (IL); MARSAULT, Boaz, 31905 Haifa (IL)
(74) Representative: Boden, Keith McMurray
(86) International application number: PCT/IB2022/052511
(87) International publication number: WO 2022/200964

(56) References cited:
- US-A1- 2019 133 673
- US-A1- 2020 171 297

## Description

### BACKGROUND

Tumor treating fields (TTFields) are low intensity alternating electric fields within the intermediate frequency range, which may be used to treat tumors as described in U.S. Patent No. 7,565,205. TTFields are induced non-invasively into the region of interest by transducers placed on the patient's body and applying AC voltages between the transducers. Conventionally, transducers used to generate TTFields include a plurality of ceramic disks. One side of each ceramic disk is positioned against the patient's skin, and the other side of each disk has a conductive backing. Electrical signals are applied to this conductive backing, and these signals are capacitively coupled into the patient's body through the ceramic disks. Conventional transducer designs include rectangular arrays of ceramic disks aligned with each other in straight rows and columns and attached to the subject's body via adhesive.

US-A-2020/0171297 discloses a transducer array for use in tumor-treating fields therapy, which has features that increase its flexibility and adhesion to a patient's skin, including a branching configuration and a correspondingly-branching top covering adhesive-backed layer.

### SUMMARY OF THE INVENTION

One aspect of the invention is directed to a transducer apparatus for delivering tumor treating fields to a subject's body according to claim 1.

Embodiments of the invention are disclosed in the dependent claims.

The above aspect of the invention is exemplary, and other aspects and variations of the invention will be apparent from the following detailed description of embodiments.

### BRIEF DESCRIPTION OF THE DRAWINGS

FIG. 1 depicts an example of transducers located on a subject's head.
FIG. 2 depicts an example of transducers located on a subject's body.
FIGS. 3A and 3B are cross-sectional views of example structures of transducers.
FIGS. 3C and 3D depict example layouts of arrays of electrode elements.
FIGS. 4A and 4B depict an example layout of electrode elements on a transducer.
FIG. 5 depicts another example layout of electrode elements on a transducer.
FIGS. 6A, 6B, and 6C depict another example layout of electrode elements on a transducer.
FIGS. 7, 8, and 9 each depict other example layouts of electrode elements on a transducer.

### DESCRIPTION OF EMBODIMENTS

This application describes exemplary transducer apparatuses for delivering TTFields to a subject's body and used to treat one or more cancers located in the subject's body.

When TTFields are applied to a subject's body, the temperature at the subject's body may increase proportionally to the induced electric field. Regulations limit the amount of current that can be driven through a transducer to an amount that keeps the measured temperature at locations on the subject's body below a threshold. Typically, the temperature at the location of the transducers on the subject's body is controlled to be below the threshold by reducing operational current driven by the transducer. This in turn becomes an over-riding limitation on the TTFields strength that can be used to treat the tumor. Thus, there is a need to safely access higher TTField strengths without exceeding the temperature threshold at the subject's skin.

The inventors discovered that, on a transducer having an array of electrode elements, electrode elements located along the edge of the array have a lower resistance to current flowing therethrough compared to electrode elements located toward the middle of the array. This can lead to higher concentrations of electric charge at points on the edge of the array in general. Further, an electrode element located at a corner or sharp bend in the array's edge will have a higher concentration than other electrode elements along the edge and in the center of the array. The tendency of a transducer to drive higher amounts of current through electrode elements at the edge (and particularly the corners) of the array is referred to herein as the "edge effect."

An uneven distribution of current through the array of a transducer due to the edge effect can lead to higher temperature zones (or "hot spots") forming at distant corners and along edges of the array. These hot spots are locations that reach the threshold temperature first and thus control the requirement to reduce the current. The generation of such hot spots limits the maximum operational current that may be driven by a transducer, and resulting TTField strength.

The inventors have now recognized that a need exists for transducers having electrode element array layouts that reduce or minimize the edge effect and allow the application of higher operating currents to the transducers. Transducers operated with increased current can induce stronger TTFields in the subject's body, leading to better patient outcomes. Transducers disclosed herein have arrays of electrode elements that reduce or minimize the edge effect.

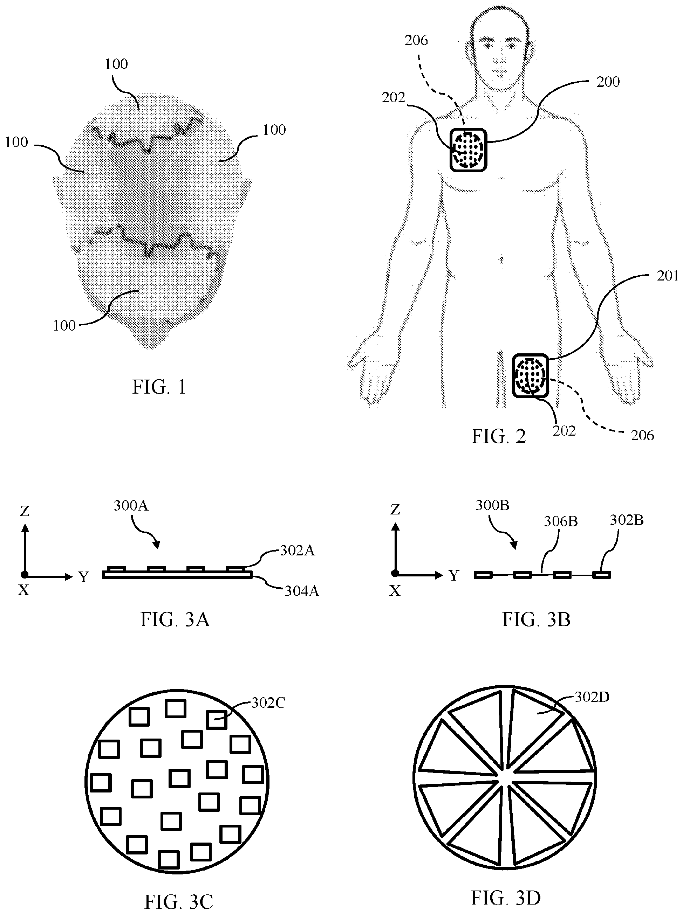

FIG. 1 depicts transducers 100 positioned on the head of a subject's body. Such an arrangement of transducers 100 on a subject's head is capable of applying TTFields to a tumor in a region of the subject's brain. Various other positions and/or orientations on the subject's head may be selected for placement of transducers. Each transducer 100 may have an array of electrode elements disposed thereon. Each transducer 100 may be placed on a subject's head with a face of the array of electrode elements facing and conforming to the subject's head.

FIG. 2 depicts transducers 200 and 201 attached to other portions (e.g., a thorax/torso and a thigh) of the subject's body. The transducers 200 and 201 may be affixed to the subject's body by applying a medically appropriate gel or adhesive onto a surface of each transducer. In other embodiments, the transducers 200 and 201 may be attached to one or more garments and held against the subject's body. Each of the transducers 200 and 201 may have an array of electrode elements 202 disposed thereon, and may be placed over the subject's body with a face of the array of electrode elements facing and conforming to the subject's body. FIG. 2 depicts the array of electrode elements 202 in both the first transducer 200 and the second transducer 201 being arranged and located within an outer perimeter (defined by a dashed line) 206. The arrays of electrode elements 202 may include any of a number of layouts as disclosed herein.

The structure of the transducers may take many forms. In FIG. 3A, the transducer 300A has a plurality of electrode elements 302A positioned on a substrate 304A. The substrate 304A is configured for attaching the transducer 300A to a subject's body. Suitable materials for the substrate 304A include, for example, cloth, foam, and flexible plastic, and also may be, or further include, a conductive medical gel. The transducer 300A may be affixed to the subject's body via the substrate 304A (e.g., via an adhesive layer and/or the conductive medical gel). The transducer may be conductive or non-conductive. FIG. 3B depicts another example of the structure of the transducer 300B. In this example, the transducer 300B includes a plurality of electrode elements 302B that are electrically and mechanically connected to one another without a substrate. In one example, electrode elements 302B are connected to each other through conductive wires 306B.

The transducers 300A and 300B may comprise arrays of substantially flat electrode elements 302A and 302B, respectively. In one example, the electrode elements 302A and 302B are ceramic disks. In another example, the electrode elements 302A and 302B are ceramic elements that are not disk-shaped. In another example, the electrode elements 302A and 302B are non-ceramic dielectric materials positioned over a plurality of flat conductors such as, for example, polymer films disposed over pads on a printed circuit board or over flat pieces of metal.

FIGS. 3A and 3B depict the transducers 300A and 300B from a direction perpendicular to a Y-Z plane defined by 3-dimensional coordinate axes shown in the figures. The electrode elements 302A and 302B may be distributed along a direction parallel to the Y-axis and/or a direction parallel to the X-axis, thereby providing an electrode element array distributed along a face of the array in a plane parallel to the X-Y plane.

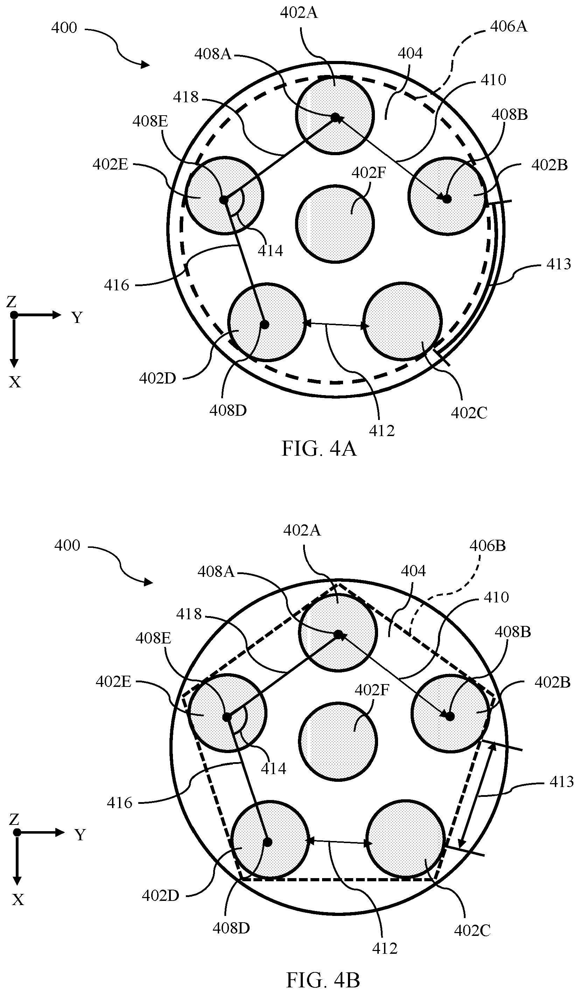

FIGS. 4A-9 each depict example layouts of electrode elements on a transducer, in accordance with disclosed embodiments. In each example, the layout is viewed from a direction perpendicular to the face (i.e., perpendicular to the X-Y plane) of the array of electrode elements. The array of electrode elements is configured to be positioned over the subject's body with this face of the array facing the subject's body. In each example layout described herein (e.g., in FIGS. 4A-9), the "array of electrode elements" comprises all electrode elements (e.g., 402A-402F in FIGS. 4A/4B) present on the transducer apparatus (e.g., 400 in FIGS. 4A/4B).

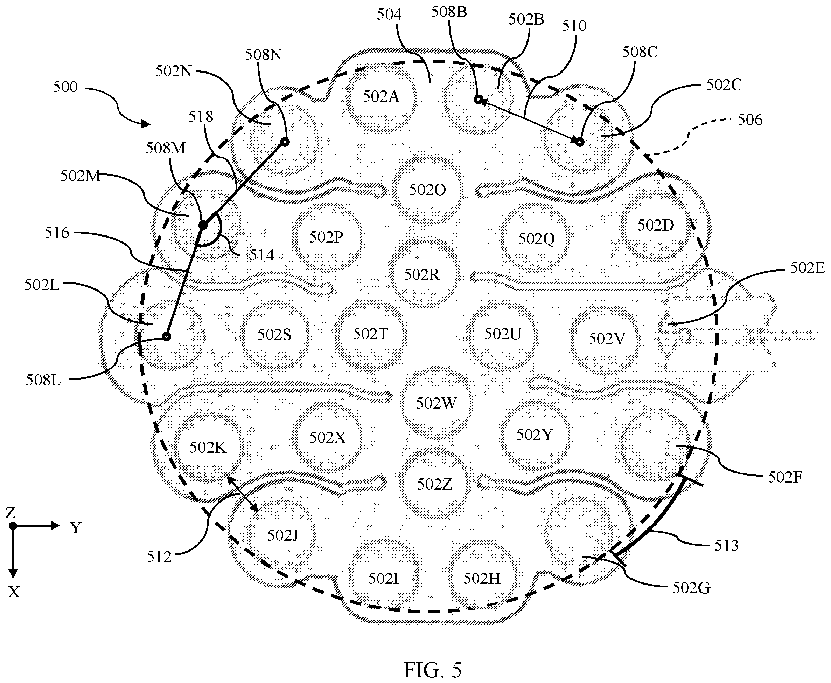

As depicted in FIGS. 4A-9, the transducer (e.g., 400 in FIGS. 4A/4B) may include a substrate (e.g., 404) on which the electrode elements are disposed. In some embodiments (e.g., FIGS. 5-8), the substrate may have cuts, slits, or perforations formed therein to facilitate placement of the substrate over rounded edges of a subject's body. Other embodiments of the transducer may not include a substrate. The disclosed electrode element layouts may be equally applied to transducers in which a substrate is present to transducers where no substrate is present.

In FIGS. 4A-9, the illustrated electrode elements (e.g., 402 in FIGS. 4A/4B) are each substantially disk-shaped. Other embodiments of the transducer may have differently shaped electrode elements. For example, as shown in FIG. 3C, the electrode elements 302C of the array may each have a square, rectangular or hexagonal shape or a substantially square, rectangular or hexagonal shape with one or more rounded corners. In another example, as shown in FIG. 3D, the electrode elements 302D of the array may each have a triangular shape, a substantially triangular shape with rounded corners, a truncated triangular shape, a substantially truncated triangular shape with rounded corners, a wedge shape, a substantially wedge shape with rounded corners, a truncated wedge shape, or a substantially truncated wedge shape with rounded corners.

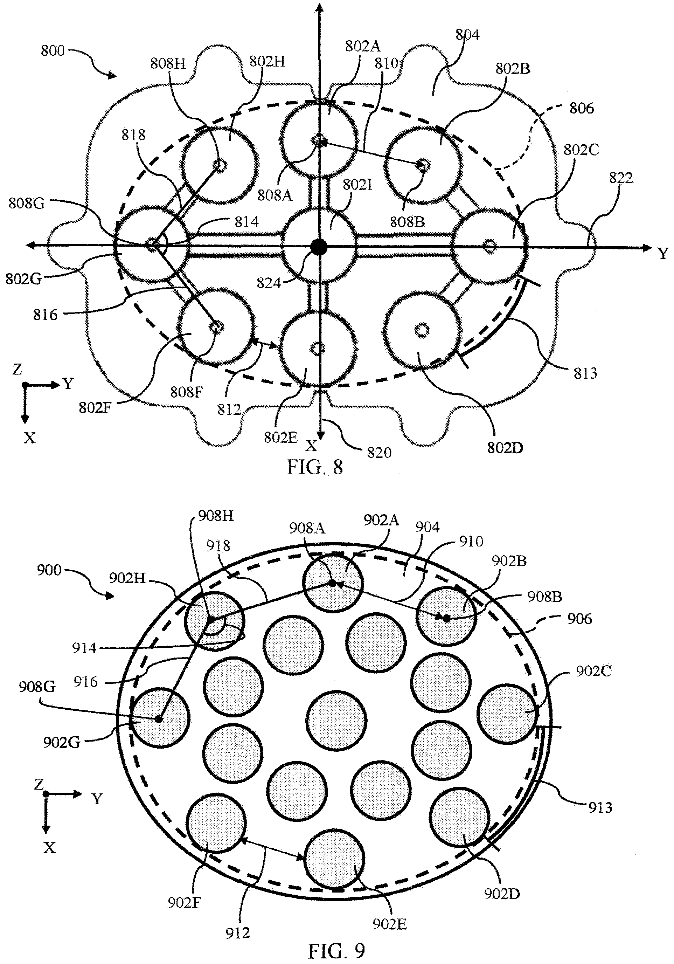

In each electrode element layout described herein (e.g., in FIGS. 4A-9), a number of the electrode elements of the array are "peripheral electrode elements." In FIGS. 4A and 4B, for example, electrode elements 402A, 402B, 402C, 402D, and 402E are peripheral electrode elements. In each figure described below, the peripheral electrode elements (e.g., 402A-E in FIGS. 4A/4B) may substantially surround all other electrode elements (e.g., 402F in FIGS. 4A/4B) of the array. The term "substantially surround" may refer to a convex shape that passes through the centroids (e.g., 408 in FIGS. 4A/4B) of all peripheral electrode elements surrounding or enclosing every other (non-peripheral) electrode element. In each figure described below, the peripheral electrode elements may define an outer perimeter (e.g., 406A/406B in FIGS. 4A/4B) of the array of electrode elements. In each embodiment described below, the array of electrode elements on a transducer includes at least five peripheral electrode elements. In some embodiments, the outer perimeter either extends through, or touches the outermost edge of, each of the peripheral electrode elements. In some transducers (e.g. FIGS. 4A, 4B, and 8) at least 80% of a total number of electrode elements in the array are peripheral electrode elements. In others (e.g. FIGS. 5-7) at least 50% of a total number of electrode elements in the array are peripheral electrode elements. In others (e.g. FIG. 9) no more than 50% of a total number of electrode elements are peripheral electrode elements.

In each electrode element layout described herein (e.g., in FIGS. 4A-9), the array of electrode elements (e.g., 402 in FIGS. 4A/4B) does not have three or more peripheral electrode elements disposed adjacent each other and aligned with each other such that a straight line passes through the centroid (e.g., 408 in FIGS. 4A/4B) of each of the three or more peripheral electrode elements. The arrays thus avoid or minimize higher current concentrations that would otherwise occur at the electrode elements at opposing ends of the straight line due to the edge effect.

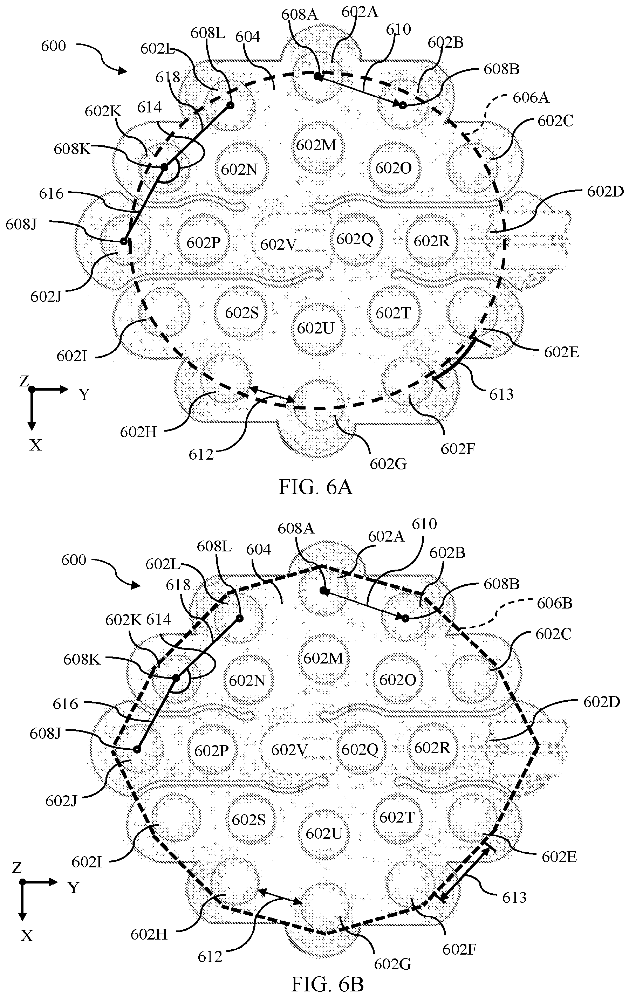

FIGS. 4A, 4B, 5, 6A-6C, 7, 8, and 9 depict transducers (400, 500, 600, 700, 800, 900) each with an example layout of electrode elements (402, 502, 602, 702, 802, 902), which may be disposed on a substrate (404, 504, 604, 704, 804, 904). The layout of electrode elements 402 is the same in FIGS. 4A and 4B. The layout of electrode elements 602 is the same in FIGS. 6A-6C.

In FIGS. 4A and 4B, the array of electrode elements comprises five peripheral electrode elements 402A-E, and one non-peripheral electrode element 402F. As such, about 83.3% of a total number of electrode elements in the array are peripheral electrode elements.

In FIG. 5, the array of electrode elements comprises fourteen peripheral electrode elements 502A-N, and twelve non-peripheral electrode element 502O-Z. As such, about 53.8% of a total number of electrode elements in the array are peripheral electrode elements.

In FIGS. 6A-6C, the array of electrode elements comprises twelve peripheral electrode elements 602A-L, and ten non-peripheral electrode elements 602M-V. As such, about 54.5% of a total number of electrode elements in the array are peripheral electrode elements.

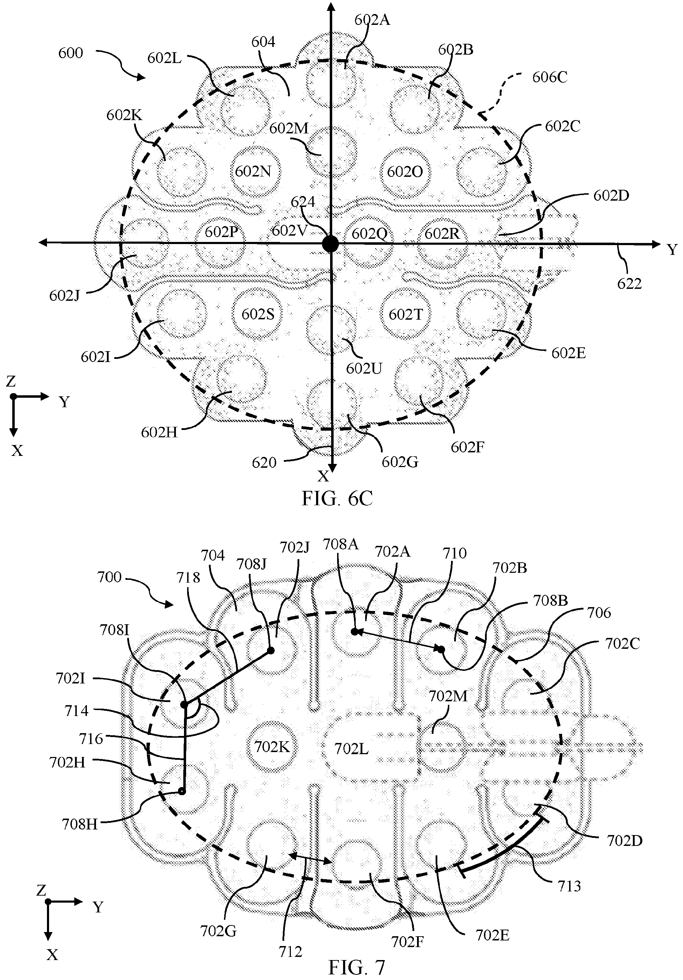

In FIG. 7, the array of electrode elements comprises ten peripheral electrode elements 702A-J, and three non-peripheral electrode elements 702K-M. As such, about 76.9% of a total number of electrode elements in the array are peripheral electrode elements.

In FIG. 8, the array of electrode elements comprises eight peripheral electrode elements 802A-H, and one non-peripheral electrode element 802I. As such, about 88.9% of a total number of electrode elements in the array are peripheral electrode elements.

In FIG. 9, the array of electrode elements comprises eight peripheral electrode elements 902A-H, and nine non-peripheral electrode elements. As such, about 47.1% of a total number of electrode elements in the array are peripheral electrode elements.

In FIGS. 4A, 4B, 5, 6B, 7, 8, and 9, an outer perimeter (406A, 406B, 506, 606B, 706, 806, and 906, respectively) is disposed along and touches an edge of at least a majority of the peripheral electrode elements. In an example, the outer perimeter (406A, 406B, 506, 606B, 706, 806, 906) may be disposed along and touch an edge of all of the peripheral electrode elements. In an example, the outer perimeter (e.g., 406A/406B in FIGS. 4A/B) may touch at least five of the electrode elements (e.g., 402A-402E) of the array, the at least five electrode elements touched by the outer perimeter being peripheral electrode elements.

An outer perimeter of the array of electrode elements may be defined in other ways as well. In FIG. 6A, for example, an outer perimeter 606A of the array may extend through at least a majority of the peripheral electrode elements (e.g., 602A-L). In an example, the outer perimeter 606A may extend through all of the peripheral electrode elements of the array. In FIGS. 4A, 4B, 5, 6B, 7, 8 and 9, the outer perimeter (406A, 406B, 506, 606B, 706, 806, 906) circumscribes the array of electrode elements.

As depicted in FIGS. 4A-9, the outer perimeter (406A, 406B, 506, 606A, 606B, 606C, 706, 806, 906) may be entirely convex in shape. The array may have a convex outer perimeter (406A, 406B, 506, 606A, 606B, 606C, 706, 806, 906) substantially tracing the array of electrode elements (402, 502, 602, 702, 802, 902). In one example, as depicted in FIGS. 4A, 5, 6A, 6C, 7, 8, and 9, the convex outer perimeter (406A, 506, 606A, 606C, 706, 806, 906) may be continuously rounded and without a straight line portion. The convex outer perimeter (406A, 506, 606A, 606C, 706, 806, 906) may be substantially circular, oval, ovaloid, ovoid, or elliptical in shape. In another example, as depicted in FIGS. 4B and 6B, the array has a convex outer perimeter (406B, 606B) substantially tracing the array of electrode elements (402, 602) with at least one straight line portion. In FIG. 4B, the convex outer perimeter 406B has a regular polygon shape or a substantially polygonal shape with rounded or curved vertices. In FIG. 6B, the convex outer perimeter 606B has an irregular polygon shape or an irregular polygonal shape with rounded or curved vertices.

In FIGS. 4A, 4B, 5, 6A, 6B, 6C, 7, 8, and 9, for each pair of adjacent peripheral electrode elements along the outer perimeter (406A, 406B, 506, 606A, 606B, 606C, 706, 806, 906) a distance between the pair of adjacent peripheral electrode elements is not more than 25% greater, or not more than 20%, 15%, or 10% greater, or, even, not more than 5% greater than a distance between any other pair of adjacent peripheral electrode elements of the array. For example, with reference to FIGS. 4A and 4B, for the pair of adjacent electrode elements 402A and 402B, the distance between these electrode elements is not more than 25% greater, or not more than 20%, 15%, or 10% greater, or, even, not more than 5% greater than any one of the distances between: electrode elements 402B and 402C; electrode elements 402C and 402D; electrode elements 402D and 402E; and electrode elements 402E and 402A.

In an example, as depicted in FIGS. 4A/B, 5, 6A/B, 7, 8, and 9, the "distance" between each pair of adjacent peripheral electrode elements may be a distance (410, 510, 610, 710, 810, 910) from a centroid (e.g., 408A, 508B, 608A, 708A, 808A, 908A) of a first peripheral electrode element (e.g., 402A, 502B, 602A, 702A, 802A, 902A) to a centroid (e.g., 408B, 508C, 608B, 708B, 808B, 908B) of a second adjacent peripheral electrode element (e.g., 402B, 502C, 602B, 702B, 802B, 902B). In another example, as depicted in FIGS. 4A/B, 5, 6A/B, 7, 8, and 9, the "distance" between each pair of adjacent peripheral electrode elements may be a shortest distance (412, 512, 612, 712, 812, 912) from an edge of a first peripheral electrode element (e.g., 402C, 502J, 602G, 702F, 802E, 902E) to an edge of a second adjacent peripheral electrode element (e.g., 402D, 502K, 602H, 702G, 802F, 902F).

As depicted in FIGS. 4A/B, 5, 6A/B, 7, 8, and 9, among peripheral electrode elements of the array touching the outer perimeter (406A/B, 506, 606A/B, 706, 806, 906), a spacing between adjacent peripheral electrode elements along the outer perimeter may have a variation of less than 25%. This spacing along the outer perimeter may be a distance (413, 513, 613, 713, 813, 913) of a line and/or arc following the outer perimeter (406A/B, 506, 606A/B, 706, 806, 906) from an outermost edge of a first peripheral electrode element (e.g., 402B, 502F, 602E, 702D, 802C, 902C) in contact with the outer perimeter to an outermost edge of a second peripheral electrode element (e.g., 402C, 502G, 602F, 702E, 802D, 902D) in contact with the outer perimeter.

As depicted in FIGS. 4A/B, 5, 6A/B, 7, 8, and 9, the peripheral electrode elements of the array may be spaced from each other along the outer perimeter (406A/B, 506, 606A/B, 706, 806, 906) with a variation in the spacing between adjacent peripheral electrode elements of less than 25%, or less than 20%, 15%, or 10%, or, even, less than 5%. The "spacing between adjacent peripheral electrode elements" may refer to the distance (410, 510, 610, 710, 810, 910) between respective centroids of adjacent electrode elements discussed above, the distance (412, 512, 612, 712, 812, 912) between adjacent electrode elements (as measured by shortest edge to edge distance) discussed above, or the distance (413, 513, 613, 713, 813, 913) following the outer perimeter (406A/B, 506, 606A/B, 706, 806, 906) discussed above.

In FIGS. 4A/B, 5, 6A/B, 7, 8, and 9, for each peripheral electrode element, an angle (414, 514, 614, 714, 814, 914) formed between the peripheral electrode element (e.g., 402E, 502M, 602K, 702I, 802G, 902H) and its two adjacent peripheral electrode elements (e.g., 402D/A, 502L/N, 602J/L, 702H/J, 802F/H, 902G/A) along the outer perimeter may be greater than 90 degrees and less than 180 degrees, the angle facing interior to the array. As depicted, the angle (414, 514, 614, 714, 814, 914) may be measured between a first line (416, 516, 616, 716, 816, 916) connecting a centroid (e.g., 408E, 508M, 608K, 708I, 808G, 908H) of the peripheral electrode element (e.g., 402E, 502M, 602K, 702I, 802G, 902H) to a centroid (e.g., 408D, 508L, 608J, 708H, 808F, 908G) of a first adjacent peripheral electrode element (e.g., 402D, 502L, 602J, 702H, 802F, 902G) and a second line (418, 518, 618, 718, 818, 918) connecting the centroid of the peripheral electrode element to a centroid (e.g., 408A, 508N, 608L, 708J, 808H, 908A) of a second adjacent peripheral electrode element (e.g., 402A, 502N, 602L, 702J, 802H, 902A). The angle (414, 514, 614, 714, 814, 914) formed between at least one peripheral electrode element and its adjacent peripheral electrode elements may be greater than 108 degrees and less than 162 degrees and/or greater than 120 degrees and less than 150 degrees.

The array of electrode elements may have symmetry with respect to one or more axes. For example, in FIGS. 6C and 8, an X-axis (620, 820) and a Y-axis (622, 822) of the array are perpendicular to each other and intersect each other at a centroid (624, 824) of the array and in the plane of the array. A first electrode element (602A, 802A) in the array is located at a first point of intersection between the X-axis (620, 820) and the outer perimeter (606C, 806); a second electrode element (602G, 802E) in the array is located at a second point of intersection between the X-axis (620, 820) and the outer perimeter (606C, 806); a third electrode element (602D, 802C) in the array is located at a first point of intersection between the Y-axis (622, 822) and the outer perimeter (606C, 806); and a fourth electrode element (602J, 802G) in the array is located at a second point of intersection between the Y-axis (622, 822) and the outer perimeter (606C, 806). As depicted in FIG. 8, the transducer 800 may include at least four additional electrode elements (802B, 802D, 802F, and 802H) that touch the outer perimeter 806. Having eight electrode elements that touch the outer perimeter 806 in this manner may help to reduce the edge effect by distributing the electrode elements 802 more evenly in the array and with a more rounded edge.

The Y-axis (622, 822) may be aligned with a largest dimension (e.g., horizontal in FIGS. 6C and 8) of the array of electrode elements (602, 802) as measured along a straight line intersecting the outer perimeter (606C, 806) in two locations. Having electrode elements at locations in which the circumscribed outer perimeter (606C, 806) intersects these axes may create symmetry in the electrode array. The symmetry along these dimensions may help to reduce the edge effect by distributing the electrode elements (602, 802) more evenly throughout the array. The outer perimeter (606C, 806) may be symmetrical with respect to the X-axis (620, 820) and/or symmetrical with respect to the Y-axis (622, 822). The array of electrode elements (602, 802) may be symmetrical with respect to the X-axis (620, 820) and may be symmetrical with respect to the Y-axis (622, 822). Although not shown in FIGS. 6C and 8, in some embodiments the array of electrode elements (602, 802) may be symmetrical with respect to the X-axis but not the Y-axis or may be symmetrical with respect to the Y-axis but not the X-axis.

Embodiments illustrated under any heading or in any portion of the disclosure may be combined with embodiments illustrated under the same or any other heading or other portion of the disclosure unless otherwise indicated herein or otherwise clearly contradicted by context.

Modifications, alterations, and changes to the described embodiments are possible without departing from the scope of the present invention defined in the claims. The present invention has the full scope defined by the language of the claims.

Embodiments illustrated under any heading or in any portion of the disclosure may be combined with embodiments illustrated under the same or any other heading or other portion of the disclosure unless otherwise indicated herein or otherwise clearly contradicted by context.

Modifications, alterations, and changes to the described embodiments are possible without departing from the scope of the present invention defined in the claims. The present invention has the full scope defined by the language of the claims.

## Claims

1. A transducer apparatus for delivering tumor treating fields to a subject's body, the transducer apparatus comprising:
an array of electrode elements (402A-F; 502A-Z; 602A-V; 702A-M; 802A-I; 902A-H), the array comprising all electrode elements (402A-F; 502A-Z; 602A-V; 702A-M; 802A-I; 902A-H) present on the transducer apparatus and being configured to be positioned over the subject's body with a face of the array facing the subject's body;
wherein, when viewed from a direction perpendicular to the face of the array:
a number of the electrode elements (402A-E; 502A-N; 602A-L; 702A-J; 802A-H; 902A-H) of the array are peripheral electrode elements defining an outer perimeter (406A; 406B; 506; 606A; 606B; 606C; 706; 806; 906) of the array, the peripheral electrode elements (402A-E; 502A-N; 602A-L; 702A-J; 802A-H; 902A-H) substantially surrounding all other electrode elements (402F; 502O-Z; 602M-V; 702K-M; 8021) of the array;
**characterised in that**
for each pair of adjacent peripheral electrode elements (402A-E; 502A-N; 602A-L; 702A-J; 802A-H; 902A-H) along the outer perimeter (406A; 406B; 506; 606A; 606B; 606C; 706; 806; 906), a distance (410, 412, 413; 510, 512, 513; 610, 612, 613; 710, 712, 713; 810, 812, 813; 910, 912, 913) between the pair of adjacent peripheral electrode elements (402A-E; 502A-N; 602A-L; 702A-J; 802A-H; 902A-H) is not more than 25% greater than a distance between any other pair of adjacent peripheral electrode elements (402A-E; 502A-N; 602A-L; 702A-J; 802A-H; 902AH); and
for each peripheral electrode element (402A-E; 502A-N; 602A-L; 702A-J; 802A-H; 902A-H), an angle (414; 514; 614; 714; 814; 914) formed between the peripheral electrode element (402A-E; 502A-N; 602A-L; 702A-J; 802A-H; 902A-H) and its two adjacent peripheral electrode elements (402A-E; 502A-N; 602A-L; 702A-J; 802A-H; 902A-H) along the outer perimeter (406A; 406B; 506; 606A; 606B; 606C; 706; 806; 906) is greater than 90 degrees and less than 180 degrees, the angle (414; 514; 614; 714; 814; 914) facing interior to the array.

2. The transducer apparatus of claim 1, wherein the outer perimeter (406A; 406B; 506; 606A; 606B; 606C; 706; 806; 906) either extends through, or touches the outermost edge of, each of the peripheral electrode elements (402A-E; 502A-N; 602A-L; 702A-J; 802A-H; 902A-H).

3. The transducer apparatus of claim 1, wherein at least 50% of a total number of electrode elements (402A-F; 502A-Z; 602A-V; 702A-M; 802A-I; 902A-H) in the array are peripheral electrode elements (402A-E; 502A-N; 602A-L; 702A-J; 802A-H; 902A-H).

4. The transducer apparatus of claim 1, wherein at least 80% of a total number of electrode elements (402A-F; 502A-Z; 602A-V; 702A-M; 802A-I; 902A-H) in the array are peripheral electrode elements (402A-E; 502A-N; 602A-L; 702A-J; 802A-H; 902A-H).

5. The transducer apparatus of any one of the preceding claims, wherein the array comprises at least five peripheral electrode elements (402A-E; 502A-N; 602A-L; 702A-J; 802A-H; 902A-H).

6. The transducer apparatus of any one of the preceding claims, wherein, for each pair of adjacent peripheral electrode elements (402A-E; 502A-N; 602A-L; 702A-J; 802A-H; 902A-H) along the outer perimeter (406A; 406B; 506; 606A; 606B; 606C; 706; 806; 906), the distance (410, 412, 413; 510, 512, 513; 610, 612, 613; 710, 712, 713; 810, 812, 813; 910, 912, 913) between the pair of adjacent peripheral electrode elements (402A-E; 502A-N; 602A-L; 702A-J; 802A-H; 902A-H) is not more than 10% greater than the distance between any other pair of adjacent peripheral electrode elements (402A-E; 502A-N; 602A-L; 702A-J; 802A-H; 902A-H).

7. The transducer apparatus of any one of the preceding claims, wherein, for each distance (410, 412, 413; 510, 512, 513; 610, 612, 613; 710, 712, 713; 810, 812, 813; 910, 912, 913) between the pair of adjacent peripheral elements (402A-E; 502A-N; 602A-L; 702A-J; 802A-H; 902A-H) along the outer perimeter (406A; 406B; 506; 606A; 606B; 606C; 706; 806; 906), the distance (410, 412, 413; 510, 512, 513; 610, 612, 613; 710, 712, 713; 810, 812, 813; 910, 912, 913) is at least one of:
a distance (410; 510; 610; 710; 810; 910) from a centroid (408A; 508B; 608A; 708A; 808A; 908A) of a first peripheral electrode element (402A-E; 502A-N; 602A-L; 702A-J; 802A-H; 902A-H) to a centroid (408B; 508C; 608B; 708B; 808B; 908B) of a second adjacent peripheral electrode element (402A-E; 502AN; 602A-L; 702A-J; 802A-H; 902A-H); or
a shortest distance (412; 512; 612; 712; 812; 912) from an edge of a first peripheral electrode element (402A-E; 502A-N; 602A-L; 702A-J; 802A-H; 902A-H) to an edge of a second adjacent peripheral electrode element (402A-E; 502A-N; 602A-L; 702A-J; 802A-H; 902A-H).

8. The transducer apparatus of any one of the preceding claims, wherein the angle (414; 514; 614; 714; 814; 914) formed between at least one peripheral electrode element (402A-E; 502A-N; 602A-L; 702A-J; 802A-H; 902A-H) and its two adjacent peripheral electrode elements (402A-E; 502A-N; 602A-L; 702A-J; 802A-H; 902A-H) along the outer perimeter (406A; 406B; 506; 606A; 606B; 606C; 706; 806; 906) is between 108 degrees and 162 degrees.

9. The transducer apparatus of any one of the preceding claims, wherein, for each peripheral electrode element (402A-E; 502A-N; 602A-L; 702A-J; 802A-H; 902A-H), the angle (414; 514; 614; 714; 814; 914) is measured between a first line (416; 516; 616; 716; 816; 916) connecting a centroid (408E; 508M; 608K; 708I; 808G; 908H) of the peripheral electrode element (402A-E; 502A-N; 602A-L; 702A-J; 802A-H; 902A-H) to a centroid (408D; 508L; 608J; 708H; 808F; 908G) of a first adjacent peripheral electrode element (402A-E; 502A-N; 602A-L; 702A-J; 802A-H; 902A-H) and a second line (418; 518; 618; 718; 818; 918) connecting the centroid (408E; 508M; 608K; 7081; 808G; 908H) of the peripheral electrode element (402A-E; 502A-N; 602A-L; 702A-J; 802A-H; 902A-H) to a centroid (408A; 508N; 608L; 708J; 808H; 908A) of a second adjacent peripheral electrode element (402A-E; 502A-N; 602A-L; 702A-J; 802A-H; 902A-H).

10. The transducer apparatus of any one of the preceding claims, wherein the outer perimeter (406A; 406B; 506; 606A; 606B; 606C; 706; 806; 906) extends through at least a majority of the peripheral electrode elements (402A-E; 502AN; 602A-L; 702A-J; 802A-H; 902A-H) or is disposed along and touches an outermost edge of at least a majority of the peripheral electrode elements (402A-E; 502A-N; 602A-L; 702A-J; 802A-H; 902A-H).

## Patentansprüche

1. Wandlereinrichtung zur Abgabe von Tumorbehandlungsfeldern an den Körper eines Subjekts, wobei die Wandlereinrichtung Folgendes umfasst:
eine Anordnung von Elektrodenelementen (402A-F; 502A-Z; 602A-V; 702A-M; 802A-I; 902A-H), wobei die Anordnung alle Elektrodenelemente (402A-F; 502A-Z; 602A-V; 702A-M; 802A-I; 902A-H) umfasst, die an der Wandlereinrichtung vorhanden sind, und so konfiguriert ist, dass sie über dem Körper des Subjekts positioniert werden kann, wobei eine Fläche der Anordnung dem Körper des Subjekts zugewandt ist;
wobei, aus einer Richtung, senkrecht zur Fläche der Anordnung, betrachtet, eine Anzahl der Elektrodenelemente (402A-E; 502A-N; 602A-L; 702A-J; 802A-H; 902A-H) der Anordnung periphere Elektrodenelemente sind, die einen äußeren Umfang (406A; 406B; 506; 606A; 606B; 606C; 706; 806; 906) der Anordnung definieren, wobei die peripheren Elektrodenelemente (402A-E; 502A-N; 602A-L; 702A-J; 802A-H; 902A-H) im Wesentlichen alle anderen Elektrodenelemente (402F; 502O-Z; 602M-V; 702K-M; 8021) der Anordnung umgeben;
**dadurch gekennzeichnet, dass**
für jedes Paar benachbarter peripherer Elektrodenelemente (402A-E; 502A-N; 602A-L; 702A-J; 802A-H; 902A-H) entlang des äußeren Umfangs (406A; 406B; 506; 606A; 606B; 606C; 706; 806; 906) ein Abstand (410, 412, 413; 510, 512, 513; 610, 612, 613; 710, 712, 713; 810, 812, 813; 910, 912, 913) zwischen dem Paar benachbarter peripherer Elektrodenelemente (402A-E; 502A-N; 602A-L; 702A-J; 802A-H; 902A-H) nicht mehr als 25 % größer als der Abstand zwischen jedem anderen Paar benachbarter peripherer Elektrodenelemente (402A-E; 502A-N; 602A-L; 702A-J; 802A-H; 902A-H) ist und
für jedes periphere Elektrodenelement (402A-E; 502A-N; 602A-L; 702A-J; 802A-H; 902A-H) ein Winkel (414; 514; 614; 714; 814; 914), der zwischen dem peripheren Elektrodenelement (402A-E; 502A-N; 602A-L; 702A-J; 802A-H; 902A-H) und seinen zwei benachbarten peripheren Elektrodenelementen (402A-E; 502A-N; 602A-L; 702A-J; 802A-H; 902A-H) entlang des äußeren Umfangs (406A; 406B; 506; 606A; 606B; 606C; 706; 806; 906) gebildet wird, größer als 90 Grad und kleiner als 180 Grad ist, wobei der Winkel (414; 514; 614; 714; 814; 914) ins Innere der Anordnung zeigt.

2. Wandlereinrichtung nach Anspruch 1, wobei der äußere Umfang (406A; 406B; 506; 606A; 606B; 606C; 706; 806; 906) sich entweder durch jedes der peripheren Elektrodenelemente (402A-E; 502A-N; 602A-L; 702A-J; 802A-H; 902A-H) erstreckt oder dessen äußersten Rand berührt.

3. Wandlereinrichtung nach Anspruch 1, wobei mindestens 50 % einer Gesamtzahl von Elektrodenelementen (402A-F; 502A-Z; 602A-V; 702A-M; 802A-I; 902A-H) in der Anordnung periphere Elektrodenelemente (402A-E; 502A-N; 602A-L; 702A-J; 802A-H; 902A-H) sind.

4. Wandlereinrichtung nach Anspruch 1, wobei mindestens 80 % einer Gesamtzahl von Elektrodenelementen (402A-F; 502A-Z; 602A-V; 702A-M; 802A-I; 902A-H) in der Anordnung periphere Elektrodenelemente (402A-E; 502A-N; 602A-L; 702A-J; 802A-H; 902A-H) sind.

5. Wandlereinrichtung nach einem der vorstehenden Ansprüche, wobei die Anordnung mindestens fünf periphere Elektrodenelemente (402A-E; 502A-N; 602A-L; 702A-J; 802A-H; 902A-H) umfasst.

6. Wandlereinrichtung nach einem der vorstehenden Ansprüche, wobei, für jedes Paar benachbarter peripherer Elektrodenelemente (402A-E; 502A-N; 602A-L; 702A-J; 802A-H; 902A-H) entlang des äußeren Umfangs (406A; 406B; 506; 606A; 606B; 606C; 706; 806; 906), der Abstand (410, 412, 413; 510, 512, 513; 610, 612, 613; 710, 712, 713; 810, 812, 813; 910, 912, 913) zwischen dem Paar benachbarter peripherer Elektrodenelemente (402A-E; 502A-N; 602A-L; 702A-J; 802A-H; 902A-H) nicht mehr als 10 % größer als der Abstand zwischen jedem anderen Paar benachbarter peripherer Elektrodenelemente (402A-E; 502A-N; 602A-L; 702A-J; 802A-H; 902A-H) ist.

7. Wandlereinrichtung nach einem der vorstehenden Ansprüche, wobei, für jeden Abstand (410, 412, 413; 510, 512, 513; 610, 612, 613; 710, 712, 713; 810, 812, 813; 910, 912, 913) zwischen dem Paar benachbarter peripherer Elemente (402A-E; 502A-N; 602A-L; 702A-J; 802A-H; 902A-H) entlang des äußeren Umfangs (406A; 406B; 506; 606A; 606B; 606C; 706; 806; 906), der Abstand (410, 412, 413; 510, 512, 513; 610, 612, 613; 710, 712, 713; 810, 812, 813; 910, 912, 913) mindestens eines von Folgendem ist:
ein Abstand (410; 510; 610; 710; 810; 910) von einem Schwerpunkt (408A; 508B; 608A; 708A; 808A; 908A) eines ersten peripheren Elektrodenelements (402A-E; 502A-N; 602A-L; 702A-J; 802A-H; 902A-H) zu einem Schwerpunkt (408B; 508C; 608B; 708B; 808B; 908B) eines zweiten benachbarten peripheren Elektrodenelements (402A-E; 502A-N; 602A-L; 702A-J; 802A-H; 902A-H) oder
ein kürzester Abstand (412; 512; 612; 712; 812; 912) von einem Rand eines ersten peripheren Elektrodenelements (402A-E; 502A-N; 602A-L; 702A-J; 802A-H; 902A-H) zu einem Rand eines zweiten benachbarten peripheren Elektrodenelements (402A-E; 502AN; 602A-L; 702A-J; 802A-H; 902A-H).

8. Wandlereinrichtung nach einem der vorstehenden Ansprüche, wobei der Winkel (414; 514; 614; 714; 814; 914), der zwischen mindestens einem peripheren Elektrodenelement (402A-E; 502A-N; 602A-L; 702A-J; 802A-H; 902A-H) und seinen zwei benachbarten peripheren Elektrodenelementen (402A-E; 502A-N; 602A-L; 702A-J; 802A-H; 902A-H) entlang des äußeren Umfangs (406A; 406B; 506; 606A; 606B; 606C; 706; 806; 906) gebildet wird, zwischen 108 Grad und 162 Grad beträgt.

9. Wandlereinrichtung nach einem der vorstehenden Ansprüche, wobei, für jedes periphere Elektrodenelement (402A-E; 502A-N; 602A-L; 702A-J; 802A-H; 902A-H), der Winkel (414; 514; 614; 714; 814; 914) zwischen einer ersten Linie (416; 516; 616; 716; 816; 916), die einen Schwerpunkt (408E; 508M; 608K; 708I; 808G; 908H) des peripheren Elektrodenelements (402A-E; 502A-N; 602A-L; 702A-J; 802A-H; 902A-H) mit einem Schwerpunkt (408D; 508L; 608J; 708H; 808F; 908G) eines ersten benachbarten peripheren Elektrodenelements (402A-E; 502A-N; 602A-L; 702A-T; 802A-H; 902A-H) verbindet, und einer zweiten Linie (418; 518; 618; 718; 818; 918), die den Schwerpunkt (408E; 508M; 608K; 708I; 808G; 908H) des peripheren Elektrodenelements (402A-E; 502A-N; 602A-L; 702A-J; 802A-H; 902A-H) mit einem Schwerpunkt (408A; 508N; 608L; 708J; 808H; 908A) eines zweiten benachbarten peripheren Elektrodenelements (402A-E; 502A-N; 602A-L; 702A-J; 802A-H; 902A-H) verbindet, gemessen wird.

10. Wandlereinrichtung nach einem der vorstehenden Ansprüche, wobei sich der äußere Umfang (406A; 406B; 506; 606A; 606B; 606C; 706; 806; 906) durch mindestens einen Großteil der peripheren Elektrodenelemente (402A-E; 502A-N; 602A-L; 702A-J; 802A-H; 902A-H) erstreckt oder entlang eines äußersten Randes mindestens eines Großteils der peripheren Elektrodenelemente (402A-E; 502A-N; 602A-L; 702A-J; 802A-H; 902A-H) angeordnet ist und diesen berührt.

## Revendications

1. Appareil transducteur destiné à délivrer des champs de traitement tumoral au corps d'un sujet, l'appareil transducteur comprenant :
un réseau d'éléments d'électrode (402A-F ; 502A-Z ; 602A-V ; 702A-M ; 802A-I ; 902A-H), le réseau comprenant tous les éléments d'électrode (402A-F ; 502A-Z ; 602A-V ; 702A-M ; 802A-I ; 902A-H) présents sur l'appareil transducteur et configurés pour être positionnés sur le corps du sujet avec une face du réseau face au corps du sujet ;
dans lequel, lorsqu'il est vu d'une direction perpendiculaire à la face du réseau : un certain nombre d'éléments d'électrode (402A-E ; 502A-N ; 602A-L ; 702A-J ; 802A-H ; 902A-H) du réseau sont des éléments d'électrode périphériques définissant un périmètre extérieur (406A ; 406B ; 506 ; 606A ; 606B ; 606C ; 706 ; 806 ; 906) du réseau, les éléments d'électrode périphériques (402A-E ; 502A-N ; 602A-L ; 702A-J ; 802A-H ; 902A-H) entourant sensiblement tous les autres éléments d'électrode (402F ; 502O-Z ; 602M-V ; 702K-M ; 8021) du réseau ;
**caractérisé en ce que**
pour chaque paire d'éléments d'électrodes périphériques adjacents (402A-E ; 502AN ; 602A-L; 702A-J ; 802A-H ; 902A-H) le long du périmètre extérieur (406A ; 406B ; 506 ; 606A ; 606B ; 606C ; 706 ; 806 ; 906), une distance (410, 412, 413 ; 510, 512, 513 ; 610, 612, 613 ; 710, 712, 713 ; 810, 812, 813 ; 910, 912, 913) entre la paire d'éléments d'électrode périphériques adjacents (402A-E ; 502A-N ; 602A-L ; 702A-J ; 802A-H ; 902AH) n'est pas supérieure de plus de 25 % à la distance entre toute autre paire d'éléments d'électrode périphériques adjacents (402A-E ; 502A-N ; 602A-L ; 702A-J ; 802A-H ; 902AH) ; et
pour chaque élément d'électrode périphérique (402A-E ; 502A-N ; 602A-L ; 702A-J ; 802A-H ; 902A-H), un angle (414 ; 514 ; 614 ; 714 ; 814 ; 914) formé entre l'élément d'électrode périphérique (402A-E ; 502A-N ; 602A-L ; 702A-J ; 802A-H ; 902A-H) et ses deux éléments d'électrode périphériques adjacents (402A-E ; 502A-N ; 602A-L ; 702A-J ; 802A-H ; 902A-H) le long du périmètre extérieur (406A ; 406B ; 506 ; 606A ; 606B ; 606C ; 706 ; 806 ; 906) est supérieur à 90 degrés et inférieur à 180 degrés, l'angle (414 ; 514 ; 614 ; 714 ; 814 ; 914) faisant face à l'intérieur du réseau.

2. Appareil transducteur selon la revendication 1, dans lequel le périmètre extérieur (406A ; 406B ; 506 ; 606A ; 606B ; 606C ; 706 ; 806 ; 906) s'étend à travers ou touche le bord le plus extérieur de chacun des éléments d'électrode périphériques (402A-E ; 502AN ; 602A-L; 702A-J ; 802A-H ; 902A-H).

3. Appareil transducteur selon la revendication 1, dans lequel au moins 50 % du nombre total d'éléments d'électrode (402A-F ; 502A-Z ; 602A-V ; 702A-M ; 802A-I ; 902AH) dans le réseau sont des éléments d'électrode périphériques (402A-E ; 502A-N ; 602A-L ; 702A-J ; 802A-H ; 902A-H).

4. Appareil transducteur selon la revendication 1, dans lequel au moins 80 % du nombre total d'éléments d'électrode (402A-F ; 502A-Z ; 602A-V ; 702A-M ; 802A-I ; 902AH) dans le réseau sont des éléments d'électrode périphériques (402A-E ; 502A-N ; 602A-L ; 702A-J ; 802A-H ; 902A-H).

5. Appareil transducteur selon l'une quelconque des revendications précédentes, dans lequel le réseau comprend au moins cinq éléments d'électrode périphériques (402A-E ; 502A-N ; 602A-L; 702A-J ; 802A-H ; 902A-H).

6. Appareil transducteur selon l'une quelconque des revendications précédentes, dans lequel, pour chaque paire d'éléments d'électrode périphériques adjacents (402A-E ; 502A-N ; 602A-L; 702A-J ; 802A-H ; 902A-H) le long du périmètre extérieur (406A ; 406B ; 506 ; 606A ; 606B ; 606C ; 706 ; 806 ; 906), la distance (410, 412, 413 ; 510, 512, 513 ; 610, 612, 613 ; 710, 712, 713 ; 810, 812, 813 ; 910, 912, 913) entre la paire d'éléments d'électrode périphériques adjacents (402A-E ; 502A-N ; 602A-L ; 702A-J ; 802A-H ; 902AH) n'est pas supérieure de plus de 10 % à la distance entre toute autre paire d'éléments d'électrode périphériques adjacents (402A-E ; 502A-N ; 602A-L ; 702A-J ; 802A-H ; 902AH).

7. Appareil transducteur selon l'une quelconque des revendications précédentes, dans lequel, pour chaque distance (410, 412, 413 ; 510, 512, 513 ; 610, 612, 613 ; 710, 712, 713 ; 810, 812, 813 ; 910, 912, 913) entre la paire d'éléments périphériques adjacents (402A-E ; 502A-N ; 602A-L; 702A-J ; 802A-H ; 902A-H) le long du périmètre extérieur (406A ; 406B ; 506 ; 606A ; 606B ; 606C ; 706 ; 806 ; 906), la distance (410, 412, 413 ; 510, 512, 513 ; 610, 612, 613 ; 710, 712, 713 ; 810, 812, 813 ; 910, 912, 913) est au moins l'une des suivantes :
une distance (410 ; 510 ; 610 ; 710 ; 810 ; 910) entre le centre de gravité (408A ; 508B ; 608A ; 708A ; 808A ; 908A) d'un premier élément d'électrode périphérique (402A-E ; 502A-N ; 602A-L; 702A-J ; 802A-H ; 902A-H) et le centre de gravité (408B ; 508C ; 608B ; 708B ; 808B ; 908B) d'un second élément d'électrode périphérique adjacent (402A-E ; 502A-N ; 602A-L; 702A-J ; 802A-H ; 902A-H) ; ou
une distance minimale (412 ; 512 ; 612 ; 712 ; 812 ; 912) d'un bord d'un premier élément d'électrode périphérique (402A-E ; 502A-N ; 602A-L ; 702A-J ; 802A-H ; 902A-H) à un bord d'un second élément d'électrode périphérique adjacent (402A-E ; 502A-N ; 602A-L ; 702A-J ; 802A-H ; 902A-H).

8. Appareil transducteur selon l'une quelconque des revendications précédentes, dans lequel l'angle (414 ; 514 ; 614 ; 714 ; 814 ; 914) formé entre au moins un élément d'électrode périphérique (402A-E ; 502A-N ; 602A-L ; 702A-J ; 802A-H ; 902A-H) et ses deux éléments d'électrode périphériques adjacents (402A-E ; 502A-N ; 602A-L ; 702A-J ; 802A-H ; 902A-H) le long du périmètre extérieur (406A ; 406B ; 506 ; 606A ; 606B ; 606C ; 706 ; 806 ; 906) est compris entre 108 degrés et 162 degrés.

9. Appareil transducteur selon l'une quelconque des revendications précédentes, dans lequel, pour chaque élément d'électrode périphérique (402A-E ; 502A-N ; 602A-L ; 702A-J ; 802A-H ; 902A-H), l'angle (414 ; 514; 614; 714 ; 814 ; 914) est mesuré entre une première ligne (416 ; 516; 616 ; 716; 816 ; 916) reliant un centre de gravité (408E ; 508M ; 608K ; 7081 ; 808G ; 908H) de l'élément d'électrode périphérique (402A-E ; 502A-N ; 602A-L; 702A-J ; 802A-H ; 902A-H) à un centre de gravité (408D ; 508L ; 608J ; 708H ; 808F ; 908G) d'un premier élément d'électrode périphérique adjacent (402A-E ; 502A-N ; 602A-L ; 702A-J ; 802A-H ; 902A-H) et une seconde ligne (418 ; 518 ; 618 ; 718 ; 818 ; 918) reliant le centre de gravité (408E ; 508M ; 608K ; 7081 ; 808G ; 908H) de l'élément d'électrode périphérique (402A-E ; 502A-N ; 602A-L; 702A-J ; 802A-H ; 902A-H) à un centre de gravité (408A ; 508N ; 608L ; 708J ; 808H ; 908A) d'un second élément d'électrode périphérique adjacent (402A-E ; 502A-N ; 602A-L ; 702A-J ; 802A-H ; 902A-H).

10. Appareil transducteur selon l'une quelconque des revendications précédentes, dans lequel le périmètre extérieur (406A ; 406B ; 506 ; 606A ; 606B ; 606C ; 706 ; 806 ; 906) s'étend à travers au moins la majorité des éléments d'électrode périphériques (402A-E ; 502A-N ; 602A-L; 702A-J ; 802A-H ; 902A-H) ou est disposé le long et touche le bord le plus extérieur d'au moins la majorité des éléments d'électrode périphériques (402A-E ; 502A-N ; 602A-L; 702A-J ; 802A-H ; 902A-H).
